# EUROPEAN PATENT APPLICATION

(11) **EP 3 683 301 A1**
(43) Date of publication of application: **22.07.2020**
(21) Application number: 18894652.9
(22) Date of filing: 25.04.2018
(51) Int. Cl.: C12N 1/14, C12P 17/04, C12R 1/645

(54) **MOLD OMK-25 FOR PRODUCING DELTA-LACTONE AND APPLICATION THEREOF**

(30) Priority: 29.12.2017 CN 201711498395
(71) Applicant: Xiamen Oamic Biotechnology Co., Ltd., Xiamen City, Fujian 361000 (CN)
(72) Inventor: ZHAO, Xijing, Xiamen, Fujian 361000 (CN); XING, Chenguang, Xiamen, Fujian 361000 (CN); LIU, Wei, Xiamen, Fujian 361000 (CN); GONG, Hongcan, Xiamen, Fujian 361000 (CN)
(74) Representative: Verscht, Thomas Kurt Albert
(86) International application number: PCT/CN2018/084319
(87) International publication number: WO 2019/128006

(57) **Abstract**

Provided are mold OMK-25 for producing delta-lactone and an application thereof. Mold OMK-25 was deposited in China Center for Type Culture Collection on June 14, 2017 under a collection number of CCTCC M 2017339. Provided is a route for producing delta-lactone, comprising converting n-alkanoic acid or an ester thereof by mold OMK-25 to produce corresponding 5-hydroxyalkanoic acid or an ester thereof, and subjecting to a post-treatment to produce delta-lactone. The n-alkanoic acid or an ester thereof comprises C6-C18 alkanoic acid or an ester thereof.

## Description

### Technical Field

The invention belongs to the technical field of microorganism fermentation, the filamentous fungus *Mucor* sp. OMK-25 in particular, for the production of delta lactones, and the application of *Mucor* sp. OMK-25.

### Background of Invention

Delta lactones are used in food flavors, as premium flavorings, daily flavors and other functional additives. In the era of advocating natural products, many production methods of delta lactones were studied. From the initial plant extraction methods, such as Massoia lactone derived from massoia oil, to the current biological fermentation, biotransformation and esterase catalysis, such as the use of microorganisms to metabolize linoleic acid to delta-decalactone, esterase hydrolysis of fatty acids to delta-decalactone, and the use of yeasts to catalyze unsaturated lactones to saturated lactones, etc.

### Summary of Invention

The aim of the invention is to provide a filamentous fungus *Mucor* sp. OMK-25 for the production of delta lactones.

Another aim of the invention is to provide a production method for delta lactones.

The technical scheme of the invention is as follows:
A filamentous fungus *Mucor* sp. OMK-25 (*Mucor* sp. OMK-25) was preserved at the China Center for Type Culture Collection (CCTCC, Wuhan University, Wuhan City, Hubei Province) on June 14, 2017. The preservation number is CCTCC NO: M 2017339.

Another technical scheme of the invention is as follows:

The application of the aforementioned fungus *Mucor* sp. OMK-25 in the production of delta lactones.

A further technical scheme of the invention is as follows:
A method for producing delta lactones, wherein n-alkane acids or n-alkane acid esters are used as substrates and are fermented to delta lactones by the above fungus *Mucor* sp. OMK-25.

In a preferred embodiment of the invention, the following steps are included:
1) Strain activation: *Mucor* sp. OMK-25 is inoculated on a solid slant medium and cultured for 2 to 5 days at 28-30 °C. The above solid slant medium by mass ratio contains: 1.5-3.0% soluble starch, 0.1-1.0% dipotassium hydrogen phosphate, 0.05-0.3% sodium chloride, 0.1-1.0% yeast extract and 0.1-1.0% tryptone.
2) Seed culture: the strain obtained from step (1) is inoculated into a seed culture medium and incubated to exponential growth phase at 28-35 °C and 150-500 rpm. The initial pH of the above seed culture medium is 7.0 to 7.5. The seed culture medium by mass ratio contains: 2.0-3.5% soluble starch, 0.1-0.5% dipotassium hydrogen phosphate, 0.1-0.3% calcium carbonate and 0.1-1.0% yeast extract powder.
(3) Fermentation culture: the seed liquid obtained in step (2) is inoculated to a fermentation medium at a volume ratio of 8 to 12%. After the glucose is depleted in the fermentation medium at 30-40 °C and 200-500 rpm, n-alkane acid or n-alkane acid ester is added to the final concentration of 8-22 g/L. The pH is adjusted to 7.0-7.5, and fermentation is continued for 65-100 hours to produce a fermentation liquid containing 5-hydroxy alkanoic acid or an ester thereof. The initial pH of the above fermentation medium is 7.2 to 7.6. The fermentation medium by mass ratio contains: 2.0-5.0% glucose, 0.1-0.3% dipotassium hydrogen phosphate, 0.05-0.1% magnesium sulfate, 0.5-2.0% calcium carbonate, 0.1-1.0% yeast extract powder and 0.1-0.5% ammonium sulfate.
(4) The fermentation liquid obtained by step (3) is processed to obtain the fermentation liquid containing the corresponding delta lactones.

Further preferably, step (3) includes: the seed liquid obtained by step (2) is inoculated to the fermentation medium at a volume ratio of 10%.

Further preferably, the initial pH of the fermentation medium is 7.5.

Further preferably, step (3) includes: the n-alkane acid or n-alkane acid ester can be added once or in batches to a final concentration of 10 to 20 g/L.

Further preferably, the post-processing of step (4) includes: the pH of the fermentation liquid obtained in step (3) is adjusted to 1.0 to 2.0 with 10% sulfuric acid.

Further preferably, the post-processing of step (4) includes: the pH of the fermentation liquid obtained in step (3) is adjusted to 1.0 to 2.0 with 10% sulfuric acid, and the fermentation liquid is heated to 60-80 °C and held for 1 to 2 hours.

Further preferably, the n-alkane acid or n-alkane acid ester is n-alkane acids or n-alkane acid esters with C6 ∼ C18.

The beneficial effects of the invention:
1. The filamentous fungus *Mucor* sp. OMK-25 of the present invention can convert n-alkane acid or its ester to fermentation culture containing 5-hydroxyalkanic acid or its ester, and then produce delta lactones.
2. The invention develops a route for converting n-alkane acid or its ester into corresponding 5-hydroxyalkanic acid or its ester by *Mucor* sp. OMK-25 and then producing delta lactones by acidification or heating. The n-alkane acid or its ester includes C6-C18 alkanoic acid or its ester. The process is simple and the yield is high. It is different from other delta lactones methods in that the n-alkane acid or its ester can be industrially produced. The enantiomeric excess value (ee) of the obtained product is 95%-100%.

### Brief Description of Drawings

Fig. 1 is a GC test result diagram of the delta-decalactone standard.
Fig. 2 is a graph showing the results of GC chirality detection of the delta-decalactone racemic standard in the present invention.
Fig. 3 is a graph showing the results of GC detection of the delta-decalactone in the fermentation liquid in embodiment 1 of the present invention.
Fig. 4 is a graph showing the results of GC detection of the delta-decalactone in the fermentation liquid in embodiment 2 of the present invention.
Fig. 5 is a graph showing the results of GC chirality detection of the delta-decalactone finished product in Embodiment 2 of the present invention.
Fig. 6 is a GC test result diagram of the delta-dodecalactone standard.
Fig. 7 is a graph showing the results of GC chirality detection of the delta-dodecalactone racemic standard in the present invention.
Fig. 8 is a graph showing the results of GC detection of delta-dodecalactone in the fermentation liquid in embodiment 3 of the present invention.
Fig. 9 is a GC detection result diagram of the delta-dodecalactone in the fermentation liquid in embodiment 4 of the invention.
Fig. 10 is a graph showing the results of GC chirality detection of the delta-dodecalactone finished product in embodiment 4 of the present invention.

### Detailed Description

The technical scheme of the present invention is further explained and described through specific embodiments.

### Embodiment 1

(1) Strain activation: a full inoculation loop of liquid from the cryogenic glycerol tube of the preserved fungus *Mucor* sp. OMK-25 was uniformly spread over a sterile solid slant medium, after which the sterile solid slant medium containing the preserved fungus *Mucor* sp. OMK-25 was cultured at 30 °C for 5 days. The above solid slant medium contained: 1.5-3.0% soluble starch, 0.1-1.0% dipotassium hydrogen phosphate, 0.05-0.3% sodium chloride, 0.1-1.0% yeast extract and 0.1-1.0% tryptone.
(2) See culture: the strain from step (1) was inoculated into a 500 mL triangular flask containing 50 mL seed culture medium. The flask containing the fungus *Mucor* sp. OMK-25 and the seed culture medium was incubated at 30 °C and 180 rpm rotational speed for 16 to 24 hours to the exponential growth phase. The above seed culture medium was sterilized at 121 °C for 20 minutes, and the initial pH of the seed culture medium was 7.2. The seed culture medium by mass ratio contained: 2.0% soluble starch, 0.5% dipotassium hydrogen phosphate, 0.2% calcium carbonate, 1.0% yeast extract powder and the solvent was water.
(3) Fermentation culture: the seed liquid obtained in step (2) was inoculated into a 500 mL triangular flask containing 45 mL fermentation medium for fermentation. The mixture of the seed liquid and the fermentation medium was incubated at 35 °C and 200 rpm shaking speed for 20 to 27 hours (preferably 24 hours). After the glucose in the medium was depleted, the n-decanoic acid or n-decanoic acid ester was added to a final concentration of 10 g/L, and the pH was adjusted to 7.0-7.5. After further being fermented for 80 hours, the 5-hydroxy-n-decanoic acid or its ester was produced. The above fermentation medium was sterilized at 121 °C for 20 minutes, and the initial pH was 7.2. The fermentation medium by mass ratio contained: 2.0% glucose, 0.3% dipotassium hydrogen phosphate, 0.1% magnesium sulfate, 2.0% calcium carbonate, 1.0% yeast extract powder, 0.5% ammonium sulfate and the solvent was water.
(4) The pH of the fermentation liquid obtained in step (3) was adjusted to 1.0 to 2.0 with 10% sulfuric acid. The fermentation liquid was heated to 60-80 °C and kept for 1-2 hours to obtain the final fermentation liquid of the delta-decalactone. At the end of the fermentation, the concentration of the delta-decalactone in the final fermentation liquid was determined by GC method, specifically, the delta-decalactone standard was used for the comparison. The GC column used was RTX-1701 (30 m^{∗}0.25 µm^{∗}0.25 mm ID). The inlet temperature was 240 °C. The pressure was 82.7 kPa. The total flow rate was 58.3 mL/min. The column flow rate was 1.09 mL/min. The line speed was 27.7 cm/sec.

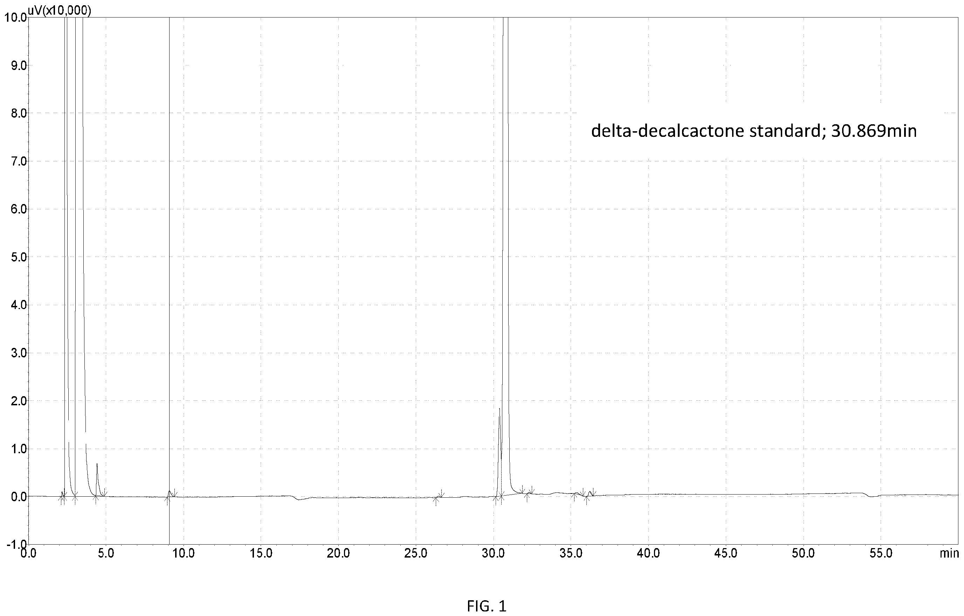

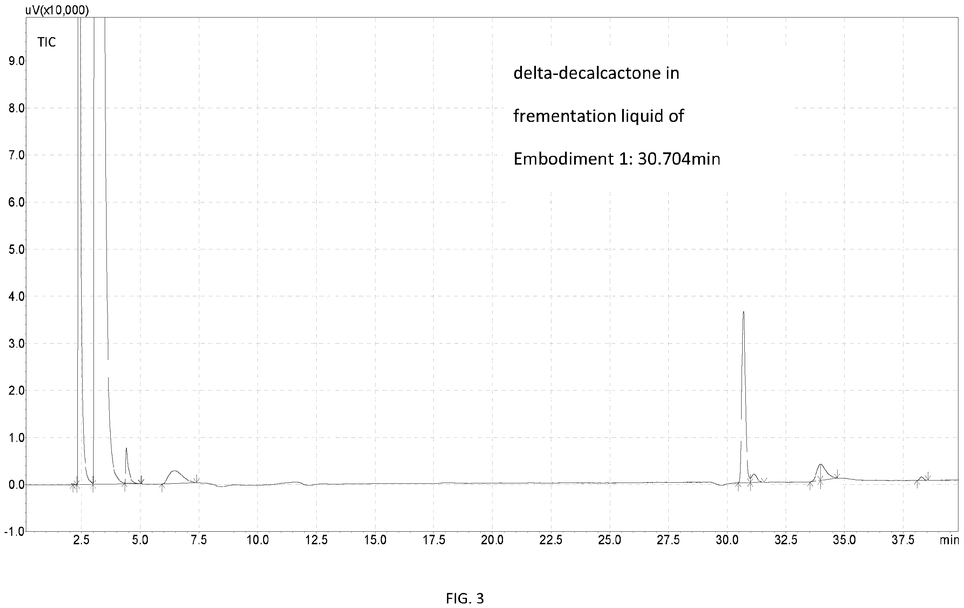

The split ratio was 50. The column temperature was 50 °C for 3 minutes. Then the temperature was raised to 230 °C at 5° C/min and kept for 20 minutes. The results were shown in Figure 1 and Figure 3. The results showed that the concentration of the delta-decalactone in the final fermentation liquid was 7.2 g/L.

### Embodiment 2

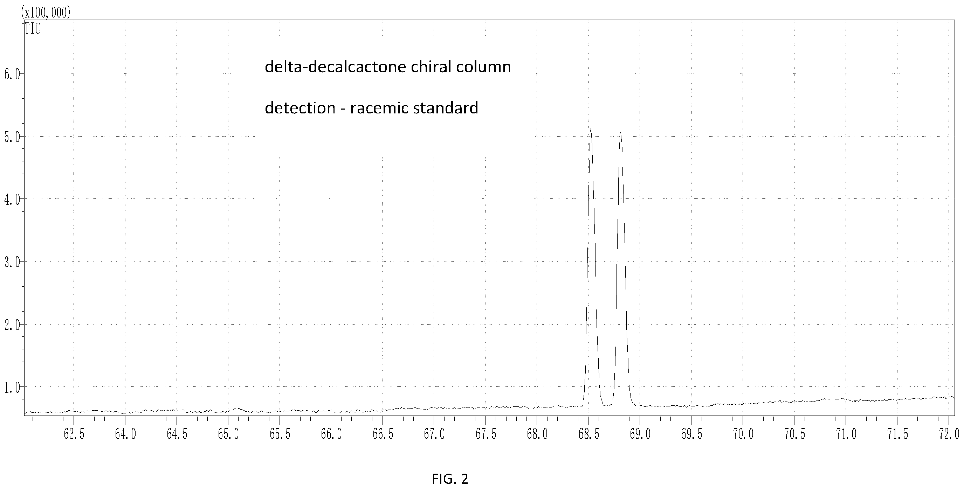

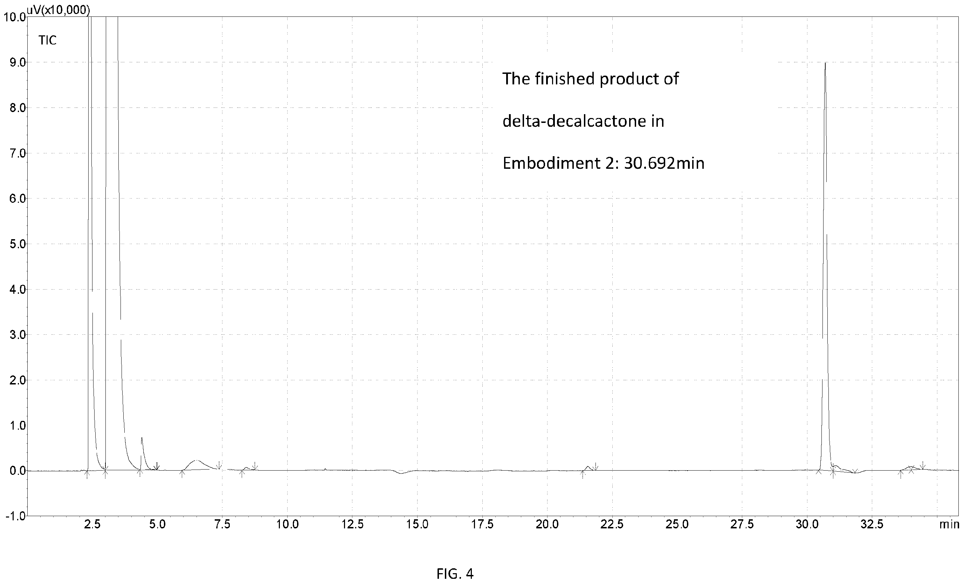

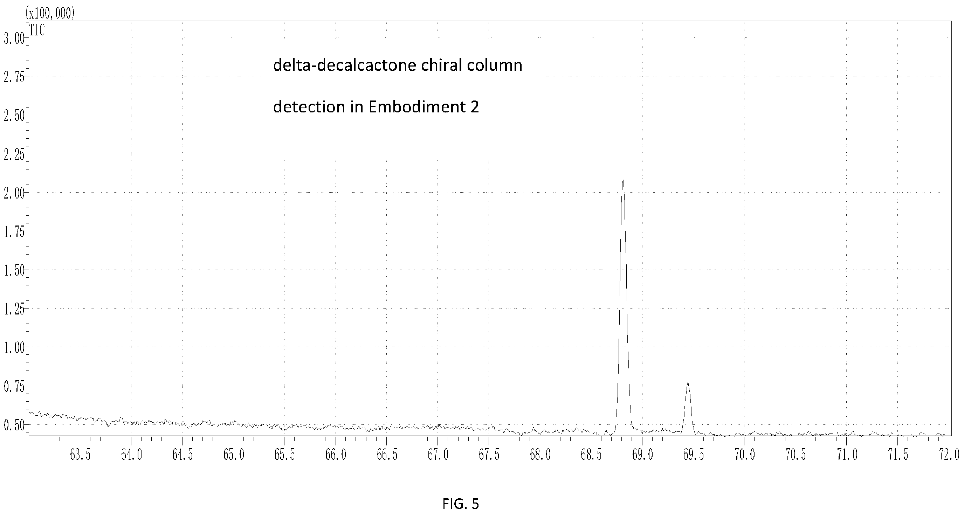

The solid slant medium, the seed medium, and the fermentation medium were the same as described in embodiment 1.
(1) A full inoculation loop of liquid from the cryogenic glycerol tube of the preserved fungus *Mucor* sp. OMK-25 was uniformly spread over the sterile solid slant medium, after which the sterile solid slant medium containing the preserved fungus *Mucor* sp. OMK-25 were cultured at 30 °C for 5 days. The strain was washed off the sloping surface with 50 mL of sterile water to form a bacterial suspension. The 50 mL bacterial suspension was inoculated under sterile conditions into a 3 L seed tank containing 1.8 L of seed culture medium for the seed liquid. The mixture of the bacterial suspension and the seed culture medium was ventilated at 300 rpm rotational speed and cultured at 30 °C for 13-18 hours to obtain the seed liquid.
(2) The prepared fermentation medium of 16.2 L was packed in a 20 L fermentation tank and sterilized at 121 °C for 20 minutes, and then 1.8 L seed liquid was added into the 20 L fermentation tank for fermentation. The fermentation temperature was 35°C. The rotation speed was 400 rpm, and the ventilation ratio was 1:0.2. Fermentation was carried on for about 10 hours until the consumption of glucose in the medium was completed. Then n-decanoic acid or n-decanoic acid ester can be added at one time or in batches to achieve a final concentration of 20 g/L. The pH of the fermentation liquid was adjusted with an acid or a base to 7.0 to 7.5. Then the fermentation liquid continued to be fermented for 60 hours to produce the fermentation liquid containing 5-hydroxy-n-decanoic acid or its ester.
(3) The pH of the fermentation liquid obtained in step (2) was adjusted to 1.0 to 2.0 with 10% sulfuric acid, the fermentation liquid was heated to 60-80 °C and kept for 1-2 hours to obtain the fermentation liquid containing delta-decalactone. At the end of the fermentation, the concentration of the delta-decalactone in the fermentation liquid was determined by GC method, specifically, the delta-decalactone standard was used for the comparison. The GC column used was RTX-1701 (30 m^{∗}0.25 µm^{∗}0.25 mm ID). The inlet temperature was 240 °C. The pressure was 82.7 kPa. The total flow rate was 58.3 mL/min. The column flow rate was 1.09 mL/min. The line speed was 27.7 cm/sec. The split ratio was 50, the column temperature was 50 °C for 3 minutes, then the temperature was raised to 230°C at 5 °C/min, and kept for 20 minutes. The results were shown in Figure 1 and Figure 4, the concentration of the delta-decalactone in the fermentation liquid was 15 g/L.
(4) The material obtained by step (3) was cooled to room temperature, extracted twice with the same amount of conventional water-insoluble organic solvents, such as ethyl acetate and butyl acetate, and the organic solvents were removed by distillation, 256 crude product of delta-decalactone was obtained. The delta-decalactone crude product was rectified to obtain 216 g of the finished product of the delta-decalactone with a purity of 99% or more. As shown in Fig. 2 and Fig. 5, the enantiomeric excess (ee) of the delta-decalactone product was detected by GC method, and its value was 100%. The GC column type of this step was RT-βDEXcst (30 m^{∗}0.25 um^{∗}0.32 mm ID). The inlet temperature was 240 °C. The pressure was 20.0 kPa. The total flow rate was 147.1 mL/min. And the column flow rate was 1.43 mL/min. The line speed was 44.0 cm/sec. The split ratio was 100.0. The column temperature was 80 °C for 5 minutes and gradually increased to 110 °C at 5 °C/min, and kept for 5 minutes at 110 °C. Then the column was heated to 130 °C at 0.5 °C/min and kept for 5 minutes. Finally, the temperature was raised to 230 °C for 50 minutes at a rate of 5 °C/min. In addition, the optical rotation of the finished product of delta-decalactone measured using a WXG-4 disc polarimeter was 56 degrees.

### Embodiment 3

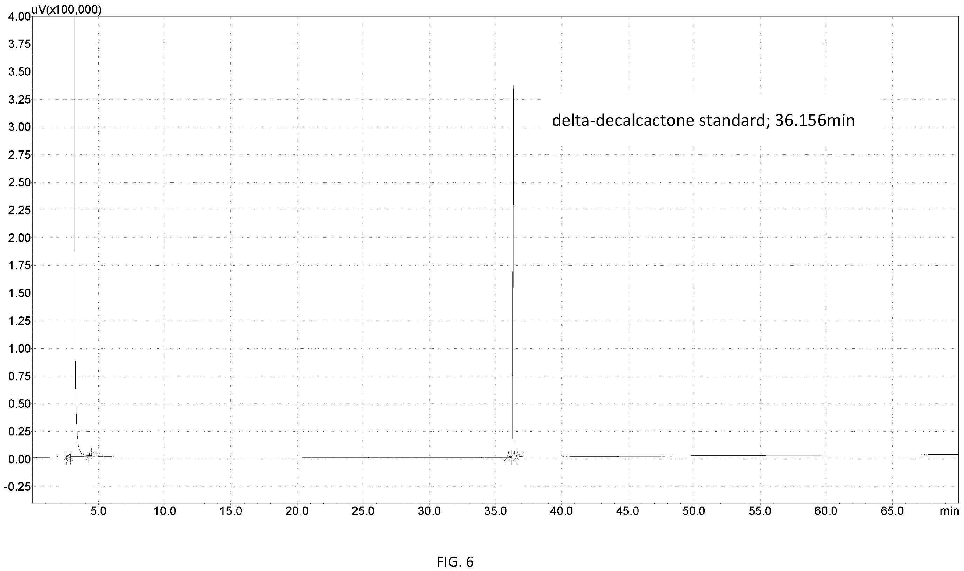

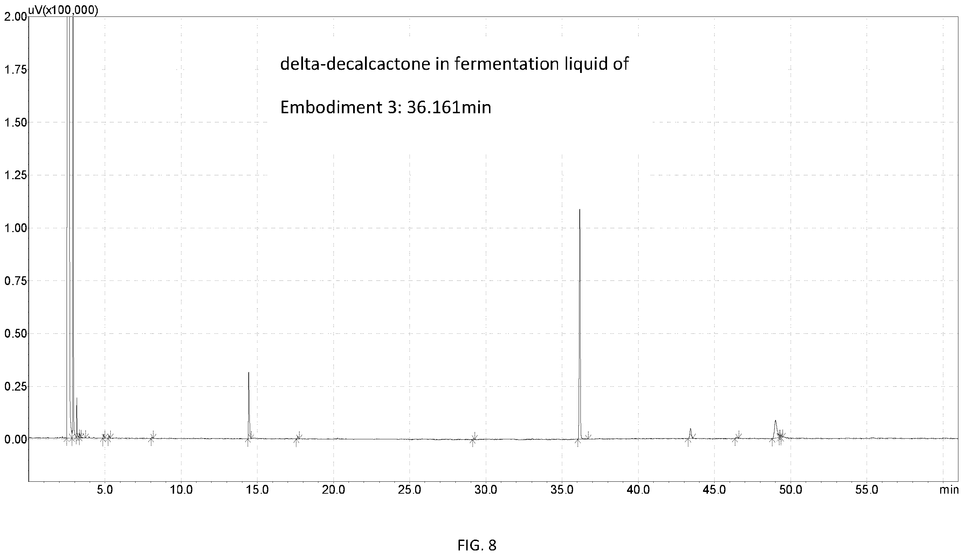

The solid slant medium, the seed medium, the fermentation medium and step (1) and step (2) were the same as described in embodiment 1.
(3) Fermentation culture: the seed liquid obtained in step (2) was inoculated into a 45 mL fermentation medium contained in a 500 mL flask. The mixture of the seed liquid and the fermentation medium was cultured at 35 °C and 200 rpm shaking speed for 20 to 27 hours (preferably 24 hours). After the glucose in the medium was depleted, the dodecanoic acid or dodecanoic acid ethyle ester was added to a final concentration of 10 g/L, and the pH was adjusted to 7.0-7.5. After further fermentation for 95 hours, the fermentation liquid containing 5-hydroxydodecanoic acid or its ethyl ester was produced.
(4) The pH of the fermentation liquid obtained in step (3) was adjusted to 1.0 to 2.0 with 10% sulfuric acid. The fermentation was heated to 60-80 °C and kept for 1-2 hours to obtain the fermentation liquid containing delta-dodecalactone. Specifically, the standard of delta-dodecalactone was selected for comparative determination. The GC column type used was RTX-1701 (30 m ^{∗} 0.25 um ^{∗} 0.25 mm ID). The inlet temperature was 240 °C. The pressure was 82.7 kPa, and the total flow was 58.3 mL/min. The column flow was 1.09 mL/min. The linear speed was 27.7 cm/sec, and the split ratio was 50. The column temperature was 50 °C for 3 minutes, and then heated up to 230 °C at 5 °C/min for 20 minutes. The results were shown in Figure 6 and Figure 8. The concentration of delta-dodecalactone in the fermentation liquid was 6.3 g/L.

### Embodiment 4

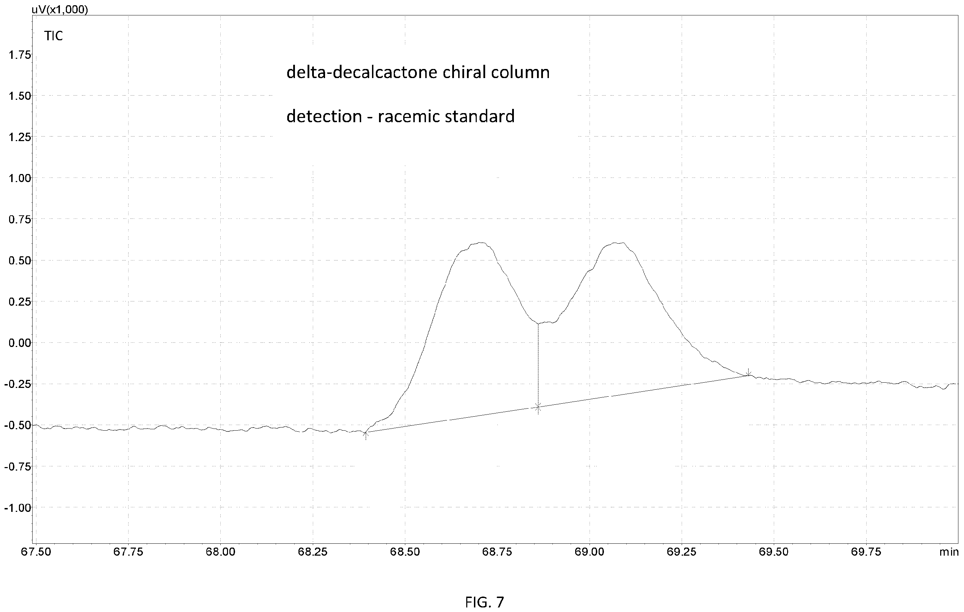

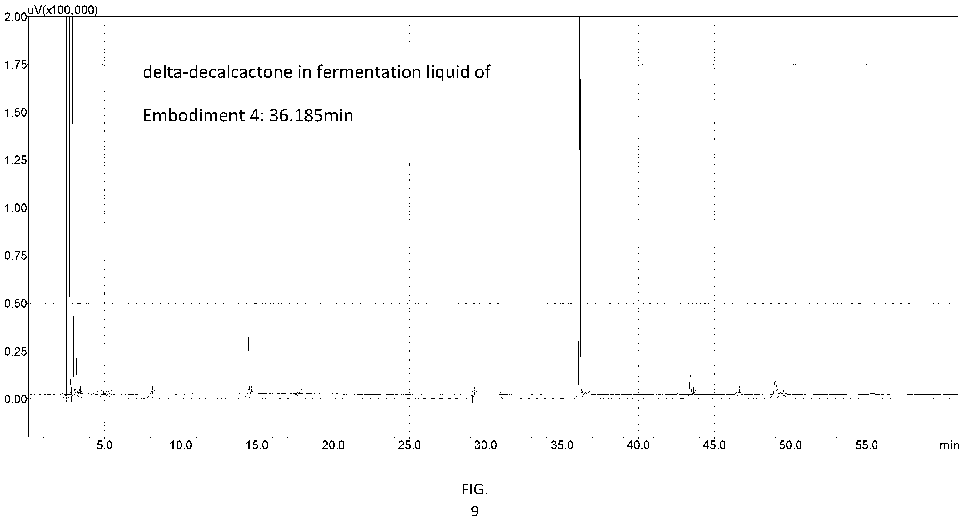

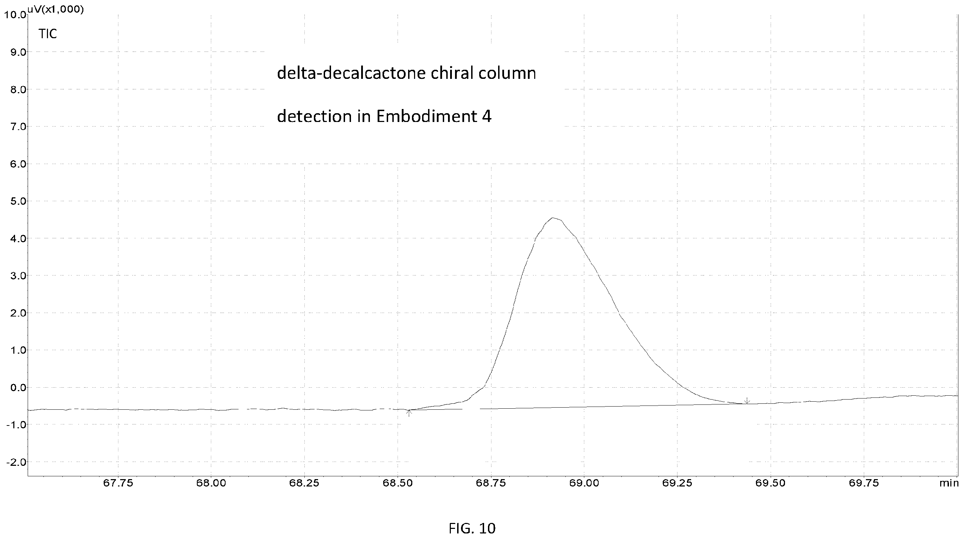

The solid slant medium and the seed medium were the same as described in embodiment 1. The fermentation medium was the same as described in embodiment 1.
(1) A full inoculation loop of liquid from the cryogenic glycerol tube of the preserved fungus *Mucor* sp. OMK-25 was uniformly coated on the sterile solid slant medium and cultured at 30 °C for 5 days. The strain was washed off the sloping surface with 50 mL of sterile water to form a bacterial suspension. The 50 mL bacterial suspension was inoculated under sterile conditions into a 3 L seed tank containing 1.8 L of seed culture medium for the seed culture. The mixture was ventilated at 300 rpm rotational speed and cultured at 30 °C for 13-18 hours to obtain the seed liquid.
(2) The prepared fermentation medium of 16.2 L was packed in a 20 L fermentation tank, and then the fermentation tank with fermentation medium was sterilized at 121 °C for 20 minutes. After that, the 1.8 L seed liquid was added into the 20 L fermentation tank for fermentation under the conditions of 35 °C fermentation temperature, 400 rpm rotational speed and 1:0.2 of ventilation ratio. About 10 hours fermentation, glucose in the medium was depleted, and n-dodecanoic acid or n-dodecanoic acid ester was added, which can be added in once or in batches. The final concentration reached 20 g/L. The pH of the fermentation liquid was adjusted to 7.0 to 7.5. After 60 hours of fermentation, the fermentation liquid containing 5-hydroxy-dodecanoic acid or its ester was produced.
(3) The pH of the fermentation liquid obtained in step (2) was adjusted to 1.0 to 2.0 with 10% sulfuric acid. the fermentation liquid was heated to 60-80 °C and kept for 1-2 hours to obtain the fermentation liquid containing delta-dodecalactone. Specifically, the delta-dodecalactone standard was used for the comparison. The GC column used was RTX-1701 (30 m^{∗}0.25 µm^{∗}0.25 mm ID). The inlet temperature was 240 °C. The pressure was 82.7 kPa. The total flow rate was 58.3 mL/min. The column flow rate was 1.09 mL/min. The line speed was 27.7 cm/sec. The split ratio was 50. The column temperature was 50 °C for 3 minutes. Then the temperature was raised to 230 °C at 5 °C/min and kept for 20 minutes at 230 °C. The results were shown in Figure 6 and Figure 9. The concentration of the delta-dodecalactone in the fermentation liquid was 12 g/L.
(4) The material obtained by step (3) was cooled to room temperature, extracted twice with the same amount of conventional water-insoluble organic solvents, such as ethyl acetate and butyl acetate, and the organic solvents were removed by distillation, 205 g crude product of delta-dodecalactone was obtained. The crude product of the delta-dodecalactone was rectified to obtain 172 g of the finished product of the delta-dodecalactone with a purity of more than 99%. As shown in Fig. 7 and Fig. 10, the enantiomer excess value (ee) of delta-dodecalactone was detected by GC method and its value was 100%. The GC column model of this step was CP-Chirasil Dex CB (25.0 m ^{∗} 0.25 um ^{∗} 0.25 mm ID). The inlet temperature was 220 °C. The pressure was 20 kPa. The total flow was 147.1 mL/min. The column flow was 1.43 mL/min. The line speed was 44.0 cm/sec. The split ratio was 100.0. The column temperature was 80 °C for 5 minutes, then the temperature was raised to 110 °C at 5 °C/min and kept for 5 minutes. Then the temperature was raised to 130 °C at 0.5 °C/min rate and kept for 5 minutes. Finally, the temperature was raised to 200 °C at 5 °C/min rate and kept for 50 minutes. In addition, the optical rotation of the fermented delta-dodecalactone was measured using a WXG-4 disc polarimeter to be 47.8 degrees.

### Embodiment 5

It will be apparent to those skilled in the art that the technical solution of the present invention can still obtain the technical effects claimed by the present invention when it is changed within the following range, and still belongs to the protection scope of the present invention:
A method for the production of delta lactones, with n-alkane acid or n-alkane acid ester as a substrate, and the above fungus *Mucor* sp. OMK-25 was used for fermentation. Preferably, the n-alkane acid or n-alkane acid ester were n-alkane acids or n-alkane acid ester with C6 ∼ C18. Specifically, the following steps were comprised:
1) Strain activation: *Mucor* sp. OMK-25 was inoculated on the solid slant medium and cultured for 2 to 5 days at 28∼30 °C; the above solid slant medium by mass ratio contained: 1.5-3.0% soluble starch, 0.1-1.0% dipotassium hydrogen phosphate, 0.05-0.3% sodium chloride, 0.1-1.0% yeast extract and 0.1-1.0% tryptone.
2) Seed culture: the strain obtained from step (1) was inoculated into the seed culture medium and cultured to exponential growth phase at 28-35 °C and 150-500 rpm rotational speed. The initial pH of the above seed culture medium was 5∼8. The seed culture medium by mass ratio contained: 2.0-3.5% soluble starch, 0.1-0.5% dipotassium hydrogen phosphate, 0.1-0.3% calcium carbonate and 0.1-1.0% yeast extract powder.
(3) Fermentation culture: the seed liquid obtained in step (2) was inoculated to the fermentation medium at a volume ratio of 8 to 12%, and after the glucose was depleted in the fermentation medium at 30 to 40 °C and 200 to 500 rpm rotational speed, n-alkanoic acid or normal acid ester was added to the concentration of 8-22 g/L, and the pH was adjusted to 7.0-7.5, and the fermentation was continued for 65-100 hours to produce the fermentation liquid containing 5-hydroxyalkanoic acid or an ester thereof. The initial pH of the above fermentation medium was 7.2 to 7.6. The fermentation medium by mass ratio contained: 2.0-5.0% glucose, 0.1-0.3% dipotassium hydrogen phosphate, 0.05-0.1% magnesium sulfate, 0.5-2.0% calcium carbonate, 0.1-1.0% yeast extract powder and 0.1-0.5% ammonium sulfate.
(4) the fermentation liquid obtained by step (3) was processed to obtain the fermentation liquid containing the corresponding delta lactones.

The above mentioned is only a better embodiment of the invention. Therefore, the scope of the invention can not be limited, that is, the equivalent changes and modifications made according to the scope and contents of the patent of the invention shall still belong to the scope covered by this invention.

## Claims

1. A filamentous fungus *Mucor* sp. OMK-25 for the production of delta lactones, wherein the preservation number of *Mucor* sp. OMK-25 is CCTCC M 2017339.

2. The application of the fungus *Mucor* sp. OMK-25 according to claim 1 in the production of delta lactones.

3. A method for the production of delta lactones, wherein n-alkane acid or n-alkane acid ester is used as a substrate and the fermentation process is by the filamentous fungus *Mucor* sp. OMK-25 according to claim 1.

4. The method for producing delta lactones according to claim 3, wherein the following steps are included:
(1) strain activation: the fungus *Mucor* sp. OMK-25 is inoculated on the solid slope medium and cultured for 2 to 5 days at 28∼30 °C, the above solid slant medium by mass ratio contains 1.5-3.0% soluble starch, 0.1-1.0% dipotassium hydrogen phosphate, 0.05-0.3% sodium chloride, 0.1-1.0% yeast extract and 0.1-1.0% tryptone;
(2) seed culture: the strain obtained from step (1) is inoculated into the seed culture medium and cultured to exponential growth phase at 28-35 °C and 150-500 rpm rotational speed, the initial pH of the seed culture medium is 7.0 to 7.5, the seed culture medium by mass ratio contains 2.0-3.5% soluble starch, 0.1-0.5% dipotassium hydrogen phosphate, 0.1-0.3% calcium carbonate and 0.1-1.0% yeast extract powder;
(3) fermentation culture: the seed liquid obtained in step (2) is inoculated into the fermentation medium at a volume ratio of 8 to 12%, and after the glucose is depleted in the fermentation medium at 30 to 40 °C and 200 to 500 rpm rotational speed, n-alkanoic acid or normal acid ester is added to the concentration of 8-22 g/L, and the pH is adjusted to 7.0-7.5, and the fermentation is continued for 65-100 hours to produce the fermentation liquid containing 5-hydroxyalkanoic acid or an ester thereof, the initial pH of the above fermentation medium is 7.2 to 7.6, the fermentation medium by mass ratio contains: 2.0-5.0% glucose, 0.1-0.3% dipotassium hydrogen phosphate, 0.05-0.1% magnesium sulfate, 0.5-2.0% calcium carbonate, 0.1-1.0% yeast extract powder and 0.1-0.5% ammonium sulfate.
(4) the fermentation liquid obtained by step (3) is processed to obtain the fermentation liquid containing the corresponding delta lactones.

5. The method for producing delta lactones according to claim 4, wherein in step (3) the seed liquid obtained by step (2) is inoculated to the fermentation medium at a volume ratio of 10%.

6. The method for producing delta lactones according to claim 4, wherein the initial pH of the fermentation medium is 7.5.

7. The method for producing delta lactones according to claim 4, wherein in step (3) the n-alkane acid or n-alkane acid ester can be added once or in batches to a final concentration of 10 to 20 g/L.

8. The method for producing delta lactones according to claim 4, wherein the post-processing of step (4) includes: the pH of the fermentation liquid obtained in step (3) is adjusted to 1.0 to 2.0 with 10% sulfuric acid.

9. The method for producing delta lactones according to claim 8, wherein the post-processing of step (4) includes: the fermentation liquid adjusted with 10% sulfuric acid in step (3) is heated to a temperature of 60 to 80 °C and kept for 1 to 2 hours.

10. The method for producing delta lactones according to any claim 3 to 9, wherein the n-alkane acid or n-alkane acid ester are n-alkane acids or n-alkane acid ester with C6 ∼ C18.
